# EUROPEAN PATENT APPLICATION

(11) **EP 2 878 311 A1**
(43) Date of publication of application: **03.06.2015**
(21) Application number: 13194676.6
(22) Date of filing: 27.11.2013
(51) Int. Cl.: A61K 47/14, A61K 47/26, A61K 47/34, A61K 47/42, A61K 47/44

(54) **Solubility Enhancement for Hydrophobic Drugs**

(71) Applicant: Freund Pharmatec Ltd., Tullamore Offaly (IE)
(72) Inventor: Ikeda, Masayuki, Co. Offaly (IE); Sivadas, Neeraj, Co. Offaly (IE); Nolan, Frank, Co. Offaly (IE)
(74) Representative: Gates, Marie Christina Esther

(57) **Abstract**

The present invention relates to improved pharmaceutical compositions of pharmaceutically active agents, having high bioavailability and to a method for preparing such compositions.

## Description

### Field of the Invention

The present invention relates to improved pharmaceutical compositions of hydrophobic drugs which have enhanced solubility and to a method for preparing such compositions.

### Background to the Invention

One of the biggest challenges facing the pharmaceutical and biotechnology industries at present is the poor solubility of new and established chemical entities. It is estimated that up to 90% on new molecular entities and 40% of existing compounds can be categorised as BCS class II or IV, which means that they show poor and variable oral bioavailability in vivo (Ref 1). Due to their low dissolution rate and poor bioavailability, hydrophobic drugs are challenging to administer and formulate.

Hydrophobic drugs may also suffer from food effects, erratic absorption and large variability in inter- and intra-patient dose response. While microemulsion preconcentrates have been used in the art to overcome some of these difficulties, they are often administered in a concentrated liquid or semi-solid form either as a drink solution or in a monolithic soft or hard elastic capsule. As is well known in the art, monolithic dosage forms have several disadvantages including dose dumping, susceptibility to food intake, local irritation, variable gastric emptying and transit. In addition, drink solutions are not as acceptable to patients, are difficult to store and may be dosed irregularly by a patient.

A multiparticulate solid microemulsion preconcentrate would overcome many of the above-described hurdles. However, whilst solid dispersions and emulsion preconcentrates have been explored for poorly soluble drugs, scale-up has proved to be a significant limitation in their development as a formulation tool.

In addition, there exists a need for patient-centric formulations to promote better treatment compliance. The multiparticulate format of the present invention allows individualised dosing and titration as opposed to the fixed dosing regimen of conventional dosage forms. Multiparticulates are easily swallowed and are therefore ideal for patients with swallowing difficulties (dysphagia) e.g. the elderly and children. Fenofibrate is a hydrophobic, lipid-regulating agent used in the treatment of adult endogenous hyperlipidaemia, hypercholesterolaemia and hypertriglyceridaemia. The chemical name for fenofibrate is 2-(4-(4-chlorobenzoyl)phenoxy)-2-methyl-propionate The active metabolite of fenofibrate is its hydrolyzed acid derivative, fenofibric acid. Treatment with fenofibrate leads to reductions in total cholesterol, low-density lipoprotein cholesterol, apolipoprotein B, total triglycerifes and triglyceride rich lipoprotein. Furthermore, an increase in the level of high-density lipoprotein (so called good cholesterol) and apoproteins apoA-I and apoA-II is observed on treatment with fenofibrate.

Fenofibrate is currently marketed as tablets under the trade names Tricor™, Fenoglide^{®} and Triglide^{®} and as capsules under the trade names Antara^{®} and Lipofen^{®}. Fenofibrate is poorly soluble in water and consequently has limited bioavailability. The drug has poor solubility in gastrointestinal fluid and consequently is poorly absorbed. Ibuprofen (iso-butyl-propanoic-phenolic acid) is a non-steroidal anti-inflammatory drug (NSAID) used for pain relief, fever reduction, and for reducing swelling. It has an antiplatelet effect, which is relatively mild and short-lived compared with aspirin or prescription antiplatelet drugs. In general, ibuprofen also has a vasodilation effect. Ibuprofen is available under a variety of trademarks, such as Motrin, Nurofen, Advil, and Nuprin.

Nonsteroidal anti-inflammatory drugs such as ibuprofen work by inhibiting the enzyme cyclooxygenase (COX), which converts arachidonic acid to prostaglandin H₂ (PGH₂). PGH₂, in turn, is converted by other enzymes to several other prostaglandins (which are mediators of pain, inflammation, and fever) and to thromboxane A₂ (which stimulates platelet aggregation, leading to the formation of blood clots).

Like aspirin and indomethacin, ibuprofen is a nonselective COX inhibitor, in that it inhibits two isoforms of cyclooxygenase, COX-1 and COX-2. The analgesic, antipyretic, and anti-inflammatory activity of NSAIDs appears to operate mainly through inhibition of COX-2, whereas inhibition of COX-1 would be responsible for unwanted effects on the gastrointestinal tract.

Ibuprofen is only very slightly soluble in water. Less than 1 mg of ibuprofen dissolves in 1 ml water (< 1 mg/ml).

There are many other poorly soluble pharmaceutically active agents which could be formulated in accordance with the present invention. These include benzocaine, chlorambucil, cyclophosphamide, flurazepam, ketoprofen, lidocaine, nicorandil, oxprenolol, piribedil, pirprofen, suloctidil, tropinone, trimipramine, trimethadione, diethylcarbamazine, cyclandelate, quinine, scopolamine, promethazine, triprolidine, gemfibrozil, dinoprostone, etomidate, trimeprazine, isosorbide dinitrate, bleomycin, thioridazine, mitotane, chlorphenesin, allylestrenol, ethambutol, carisoprodol, benzocaine, maprotilin and ethotoin. The invention would also be suitable for use with new molecular entities that have poor solubility.

Bioavailability is the degree to which an active ingredient, after administration becomes available to the target tissue. Poor bioavailability poses significant problems in the development of pharmaceutical compositions. Active ingredients that are poorly soluble in aqueous media often have insufficient dissolution and consequently have poor bioavailability within an organism after oral administration. If solubility is low there may be incomplete and/or erratic absorption of the drug on either an intra-patient or inter-patient basis. In order to circumvent this disadvantage, the administration of multiple therapeutic doses is often necessary.

In recent years, focus in formulation laboratories for improving the bioavailability of hydrophobic pharmacologically active ingredients has been upon reducing particle size. The rate of dissolution of a particulate drug can be increased, by decreasing particle size, through an effective increase in surface area.

Considerable effort has been made to develop methods for controlling drug particle size in pharmaceutical compositions. For example, in order to improve the rate of dissolution of fenofibrate, a wide variety of formulation methods have been employed, including micronization of the active principle, addition of a surfactant and co-micronization of fenofibrate with a surfactant.

US4961890 to Boyer, describes fenofibrate granules, in which fenofibrate is micronized in order to increase its bioavailability. Each fenofibrate granule comprises an inert core, a layer based on fenofibrate and a protective outer layer. The crystalline fenofibrate particles are less than 30 µm in diameter. The binder used is a water soluble polymer, for example polyvinylpyrrolidine, and constitutes an inert water soluble matrix in which the micronized fenofibrate is suspended. The quantity of binder used is such that the amount of fenofibrate released after stirring in a galenical preparation for 1 hour is at least 65%. No examples quantifying specifically, the release of fenofibrate in aqueous media are provided.

In the prior art, the problem of water-insoluble pharmaceutically active substances has been addressed by formulating the actives as micron and sub-micron sized particles in water or as a suspension in an aqueous environment. However these particles tend to grow over time and it is difficult to remove water from them to convert them to solid dosage forms. Alternative solutions include formulation in non-aqueous media, or in biocompatible oils which are then dispersed in water using surfactants to produce oil-in -water emulsions, or the drugs may be dissolved in water-miscible organic solvents or in mixtures of oils and surfactants.

Other solutions to the problem include specific solutions for particular actives. US patent No. 6,306,434 discloses a composition comprising cyclosporin which has low bioavailibility due to its poor aqueous solubility. The composition is a solid-state microemulsion which comprises a solidified product which consists essentially of a cyclosporin microemulsion dispersed in an enteric carrier. The carrier is typically an enteric polymer.

European patent No. 1,214,059 discloses a composition comprising water-insoluble biologically active substances dispersed in a non-aqueous carrier which comprises a non-aqueous medium in which the active is either not soluble or is poorly soluble, a surfactant which in turn comprises at least one phospholipid surfactant which is soluble in the medium, but at least a portion of which adsorbs to the surface of the drug particles, and up to 10% of at least one hydrophilic substance which provides a self-dispersing property to the composition.

US patent No. 5,952,004 discloses a composition which comprises an oil-in -water emulsion which in turn comprises a discontinuous hydrophobic phase, a continuous aqueous hydrophilic phase and at least one surfactant selected from poloxamer 124, a polyglycolised glyceride, sorbitan laurate and polyoxyethylene (20) sorbitan monooleate, for dispersing the hydrophobic phase in the hydrophilic phase. WO0016749 discloses a method for preparing novel galenic formulations of fenofibrate with improved bioavailability after oral administration consisting of (a) micronizing fenofibrate; (b) granulating the fenofibrate in the presence of a liquid medium comprising a surfactant, water and water-miscible alcohol; and (c) drying the resulting granular material.

In WO2004028506, pharmaceutical compositions of fenofibrate with high bioavailability after oral administration are disclosed. The immediate release fenofibrate composition comprises an inert hydro-insoluble carrier with at least one layer containing micronized fenofibrate, a hydrophilic polymer and a surfactant; and optionally one or several outer phases or layers.

Curtet et al. in US4895726 proposes improving the bioavailability of fenofibrate by co-micronizing it with a solid surfactant such as sodium lauryl-sulphate, wherein the mean particle size of said co-micronized mixture is less than 15 µm. The co-micronizate is then granulated by wet granulation in order to improve the flow capacities of the powder and to facilitate the transformation into gelatin capsules.

In US5882680, β-carotene is added to a middle chain length fatty acid (MCT); the resulting mixture is emulsified to produce a dispersion, which is subsequently homogenised to form a suspension. The suspension then enters a device for the manufacture of seamless capsules, namely a "Spherex" device (manufactured by Freund Industrial Co., Ltd.), the suspension is heated to 35 °C and forms an encapsulating liquid which is subsequently enveloped by an outer shell forming liquid at 70 °C composed of an aqueous solution of gelatin and sorbitol. The seamless capsule is formed when the encapsulating liquid is passed through the inner tube of series of coaxial tubes and simultaneously the outer shell liquid is passed through the outer tube of the coaxial tubing arrangement, the resulting droplets that form enter a hardening liquid of MCT cooled to 9 °C.

Other efforts to improve the solubility of fenofibrate include producing drug particles with an effective particle size of less than about 2000nm (see US patent nos. 7276249 and 7320802). Other attempts to improve solubility involve use of a crystalline drug substance which has a non-cross-linked surface modifier adsorbed onto its surface which maintains an effective particle size of less than about 400nm (see US patent no. 5145684).

Notwithstanding the state of the art there remains a need for alternative formulations for poorly soluble drugs to improve their bioavailability.

### OBJECT OF THE INVENTION

It is an object of the invention to provide an improved formulation for poorly water soluble or water insoluble active substances, which has improved bioavailability. Accordingly, a further object is to provide a novel composition, comprising poorly water soluble or water insoluble actives which have enhanced dissolution and absorption profiles.

### SUMMARY OF THE INVENTION

According to the present invention, there is provided a pharmaceutical composition comprising:
(a) a poorly soluble pharmaceutical agent;
(b) a hydrophobic component,
(c) a carrier; and
(d) a surfactant.

The poorly soluble pharmaceutical agent may have a melting point of up to 100°C.

A co-melt of the poorly soluble pharmaceutical agent with a hydrophobic component, a carrier and a surfactant is introduced as droplets into a cold hardening liquid. This rapid or quench cooling of the molten drug converts it into an amorphous state. Amorphous materials have higher free energy than their crystalline counterparts and as a result exhibit higher apparent solubility and faster dissolution rates. This inturn can lead to higher bioavailability of poorly-soluble drugs whose absorption is dissolution-rate limited. The final composition of the invention is a solid preconcentrate that upon oral intake, forms en emulsion (e.g. a microemulsion) when exposed to gastro-intestinal fluids. The invention functions by causing the amorphous drug to stay dissolved in the lipid or hydrophobic phase of the emulsion and/or in the micellar phase of the surfactant, thereby enhancing drug absorption and bioavailability.

Suitable pharmaceutical agents include fenofibrate (m.p 79-82°C), benzocaine (m.p. 88-90°C, chlorambucil (m.p. 64-66°C), cyclophosphamide (m.p. 41-45°C), flurazepam (m.p. 77-82°C), ketoprofen (m.p. 94°C), lidocaine (m.p. 68-69°C), nicorandil (m.p. 92-93°C), oxprenolol (m.p.79-80°C), piribedil (m.p. 98°C), pirprofen (m.p.98-100°C), suloctidil (m.p. 62-63°C), tropinone (m.p. 41-42°C), trimipramine (m.p. 45°C), trimethadione (m.p. 46°C), diethylcarbamazine (m.p. 48°C), cyclandelate (m.p. 56°C), quinine (m.p. 57°C), scopolamine (m.p. 59°C), promethazine (m.p 60°C), triprolidine (m.p. 60°C), gemfibrozil (m.p.62°C), dinoprostone (m.p. 67°C), etomidate (m.p.67°C), trimeprazine (m.p 68°C), isosorbide dinitrate (m.p. 70°C), bleomycin (m.p 71°C), thioridazine (m.p 73°C), mitotane (m.p. 77°C), chlorphenesin (m.p. 78°C), allylestrenol (m.p. 80°C), ethambutol (m.p. 88°C), carisoprodol (m.p. 92°C), benzocaine (m.p. 92°C), maprotilin (m.p. 93°C), ethotoin (m.p. 94°C) and ibuprofen (m.p. 75-77°C). Suitably the carrier is gelatin. The gelatin may have a bloom strength of from about 80 to about 350. Preferably the bloom strength is from about 180 to 300. The gelatin may be porcine or bovine gelatin.

The hydrophobic component may be selected from the group consisting of vegetable oils (eg. corn oil, sesame oil, olive oil, peanut oil, cottonseed oil, sunflowerseed oil), animal oils (eg. omega-3 fatty acids), esterification products of vegetable fatty acids or propylene glycol including fatty acid triglycerides (eg. Miglyol 810, Crodamol GTCC, Neobee M5, Labrafac CC, Labrafac PG, Captex 355, fractionated coconut oil), fatty acid mono- and di-glycerides (eg. Peceol, Maisine 35-1, Imwitor 988, Capmul MCM). They may also be selected from long-chain fatty alcohols (eg. stearyl alcohol, cetyl alcohol, cetostearyl alcohol), sorbitan esters (Span 80, Arlacel 20), or phospholipids (eg. egg lecithin, soybean lecithin). Preferredglycerides include Maisine 35-1, Peceol, Capmul GMO, Cithrol GMO.

The surfactant may have a HLB value of 14-16. As is well known to those of skill in the art, the hydrophilic-lipophilic balance of a surfactant is a measure of the degree to which it is hydrophilic or hydrophobic which is determined by calculating values for the different regions of the molecule. An HLB value of 0 corresponds to a completely lipophilic/hydrophobic molecule, and a value of 20 corresponds to a completely hydrophilic/lipophobic molecule.

Suitable surfactants include polyoxyl 40 hydrogenated castor oil, Gelucire 44/14 and 50/13, Labrasol, Acconon MC-8, Acconon C-44, PEG-35 castor oil.

The active pharmaceutical agent may be present in an amount of from about 1 to about 15 %w/w based on the total weight of the composition. Preferably it is present in an amount of between 7 and 10 %w/w.

The weight ratio of active pharmaceutical agent to surfactant may be in the range 1 : 1.6 to 1 : 1.29.

The formulation may be an immediate release formulation.

The composition may also be formulated as seamless spheres comprising:
(a) a poorly soluble pharmaceutical agent;
(b) a hydrophobic component,
(c) a carrier; and
(d) a surfactant.

The spheres or cores may typically have a diameter in the range of 0.5 mm to 7.0 mm, preferably 1.0 mm to 2.5 mm, more preferably, 1.4 mm to 1.7 mm.

In another aspect, the invention provides a method of manufacturing the pharmaceutical composition of the present invention comprising the steps of:
(i) melting together the pharmaceutically active agent, the hydrophobic component and the surfactant at a temperature greater than the melting point of the agent to produce a solution;
(ii) dispersing gelatin in water in a ratio of 0.8 : 1 to 1.2 : 1 by weight and allowing it to swell;
(iii) adding the solution produced in step (i) to the remaining quantity of] water which is maintained at a temperature just below its boiling point to form an emulsion;
(iv) adding the swollen gelatin to the emulsion of step (iii) and allowing the gelatin to dissolve.

The resultant mixture may be processed to produce seamless, spherical beads of size 1.4-1.7mm in diameter. The mixture may be processed using a Spherex™ technology seamless spherical microcapsule manufacturing device, to produce seamless spherical microcapsules.

One aspect of the present invention involves the manufacture of spheres comprising a poorly soluble active using the process described in EP2586429.

Also provided for is a composition according to the present invention for use as a medicament.

Advantageously, the method of manufacture is a single pot process wherein fenofibrate, polyglycolized glyceride and gelatine are mixed together and processed into spheres. The method of manufacture is significantly simpler than that of alternative fenofibrate products. For example the method of manufacture of Antara®, Tricor® and Fenoglide® involve multiple steps and complex processing methods, primarily a pre-treatment of fenofibrate (e.g. size reduction) before a final dosage form is produced.

Also provided is a composition according to the present invention for use in the treatment of hyperlipidaemia or mixed dyslipidaemia, hypercholesterolaemia and hypertriglyceridaemia.

The present invention provides an improved immediate release fenofibrate formulation, which has enhanced dissolution and absorption profiles. Advantages include reduced dose dumping, less variability in absorption compared to existing formulations, and much faster release and dissolution due to processing at the melt temperature of the drug, the minicapsule formulation and increased surface area of the spheres. Furthermore, the present invention does not require the addition of disintegrants to achieve this enhanced dissolution profile.

### Brief Description of the Drawings

Embodiments of the invention will be described, by way of example only, with reference to the accompanying drawings in which:

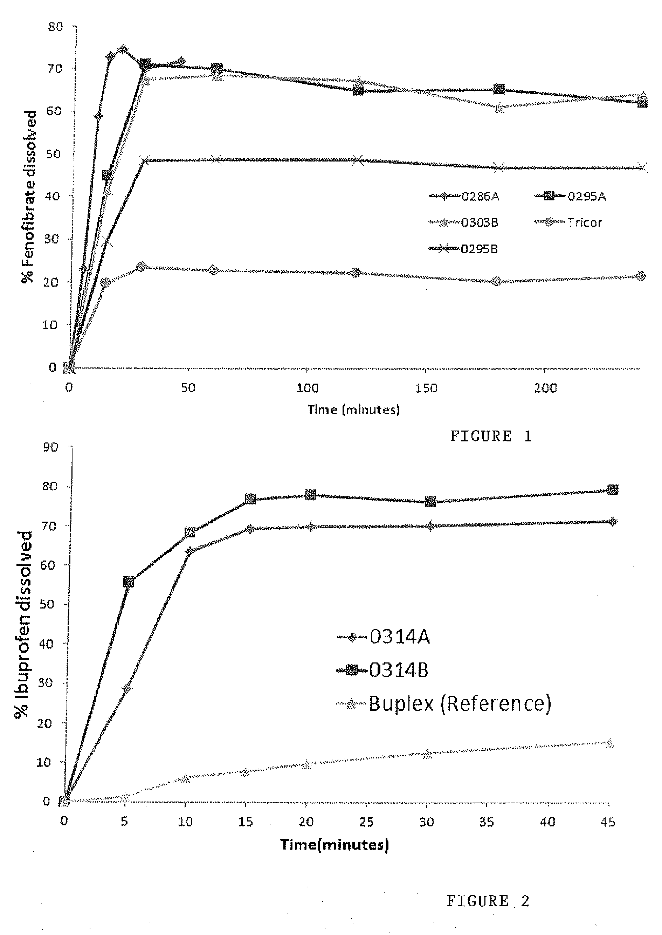

**Figure 1** shows the dissolution profiles for fenofibrate formulations in accordance with the invention; and
**Figure 2** shows the dissolution profiles for ibuprofen formulations of the present invention.

### Detailed Description of the Invention

The gelatin spheres of the present invention, incorporating fenofibrate, were manufactured according to the teachings of Freund Industrial Co. Ltd. EP2586429 the teachings of which are incorporated herein by reference. This technology is based on the principle that a laminar liquid jet can be broken into equally sized droplets by a superimposed vibration. When the droplets come in contact with a hardening liquid, they undergo gelation leading to the formation of spheres. This technique enables highspeed production of uniformly sized spheres.

Gelatin is obtained by the partial hydrolysis of collagenous material, such as skin, connective tissues, or animal bones. There are two main classes of gelatin, Type A gelatin, which is obtained from acid-processing of porcine skins and exhibits an isoelectric point between pH 7 and pH 9; and Type B gelatin which is obtained from the alkaline-processing of bovine bone and skin and exhibits an isoelectric point between pH 4.7 and pH 5.2. It will be appreciated by those skilled in the art that varying blends of gelatin are available with varying bloom strength characteristics.

In the examples disclosed below, porcine and bovine derived gelatin are used, however, the skilled person will be appreciate that other sources of gelatin are equally suitable. **Example 1:** Objective: To enhance aqueous solubility by combining a low melting point, poorly soluble drug with a system consisting of a hydrophobic component (e.g. a monoglyceride such as Maisine 35-1), a surfactant with a high HLB value (14-16) (e.g. Polyoxyl 40 hydrogenated castor oil, Tradename: Kolliphor RH40) and a carrier, preferably gelatin (either procine or bovine derived with bloom strength in the range 180-300). 2 poorly soluble drugs, Fenofibrate and Ibuprofen, having a low melting point were chosen to test the utility of the above composition. Details of the trials are provided in the table below. Two ratios of Maisine/Kolliphor, 1:1.8 and 1:2.7 were tried as shown in the last row of the table.

| | **%w/w** | | | | |
|---|---|---|---|---|---|
| Batch No. | **EXPROD -0286A, 0295A, 0303B** | **EXPROD-307A** | **EXPROD-0295B** | **EXPROD-0314A** | **EXPROD-0314B** |
| Fenofibrate | 8.99 | 8.99 | 8.99 | 0 | 0 |
| Ibuprofen | 0 | 0 | 0 | 8.99 | 8.99 |
| Maisine 35-1 (M) | 8.99 | 8.99 | 8.99 | 8.99 | 8.99 |
| Kolliphor RH40(K) | 16.374 | 16.34 | 24.51 | 16.34 | 24.51 |
| Gelatin | 65.68 | 65.68 | 57.51 | 65.68 | 57.51 |
| Drug:M:K ratio | 1:1:1.8 | 1:1:1.8 | 1:1:2.7 | 1:1:1.8 | 1:1:2.7 |

**Manufacturing Procedure:** Fenofibrate (or Ibuprofen). Maisine 35-1 and Kolliphor RH 40 were melted together at a temperature greater than the melting point of the drug (Fenofibrate: 79-81°C, Ibuprofen: 75-77°C) to obtain a clear solution. Gelatin was dispersed in water in a 1:1 ratio by weight and allowed to swell. The drug/solubiliser solution was added to the remaining quantity of water (heated at 95°C) under stirring to form an emulsion. Of the total amount of water that is weighed out for the formulation, one part is used to swell the gelatin and the remaining part is mixed with the drug and solubliser and heated up. The swollen gelatin was added to this emulsion and stirred until the gelatin dissolved. The final solid content of the system was between 27-30%w/w. The resulting mixture was used to form spherical beads of size 1.4-1.7mm using the SPHEREX™. During processing, the temperature of the drug/solubiliser/gelatin liquid was maintained above the melting point of the drug except in case of EXPROD-0307A where a lower liquid temperature (68-73°C) was used.

**Dissolution profile:** The drug release profiles of the above formulations and the reference products (TRICOR ^{®} 48mg Fenofibrated tablets and Buplex^{®} 200mg Ibuprofen tablets) was tested in biorelevant dissolution media; using Fasted State Simulated Intestinal Fluid (FaSSIF) for Tricor^{®} and Fasted State Simulated Gastric Fluid (FaSSGF) for Buplex^{®}. USP Apparatus I (Paddle) was used.
Volume of media: 900 ml;
Media temperature: 37°C.;
Paddle rotation speed: 75 rpm;
Samples taken:
5, 10, 15, 20, 30 and 45 minutes (for EXPROD-0286A, 0314A and 0314B)
15, 30, 60, 120, 180 and 240 minutes (for all other batches)
Observations: A significant increase in % drug dissolved was observed with all formulations with respect to the prior art marketed products. In case of Fenofibrate, a 1:1.8 ratio of Maisine: Kolliphor proved optimal whereas with Ibuprofen, a 1:2.7 ratio of Maisine:Kolliphor showed higher dissolution than a 1:1.8 ratio.

The words "comprises/comprising" and the words "having/including" when used herein with reference to the present invention are used to specify the presence of stated features, integers, steps or components but do not preclude the presence or addition of one or more other features, integers, steps, components or groups thereof.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination.

## Claims

1. A pharmaceutical composition comprising:
(a) a poorly soluble pharmaceutical agent;
(b) a hydrophobic component,
(c) a carrier; and
(d) a surfactant.

2. A pharmaceutical composition according to claim 1 wherein the carrier is gelatin.

3. A pharmaceutical composition according to claim 1 or 2 wherein the gelatin is either porcine or bovine derived and has a bloom strength in the range 180-300.

4. A pharmaceutical composition according to any preceding claim wherein the hydrophobic component is selected from the group consisting of vegetable oils, animal oils, esterification products of vegetable fatty acids or propylene glycol including fatty acid triglycerides, fatty acid mono- and di-glycerides, long-chain fatty alcohols, sorbitan esters, or phospholipids.

5. A pharmaceutical composition according to claim 4 wherein the hydrophobic component is a glyceride selected from the group Maisine 35-1, Peceol, Capmul GMO, and Cithrol GMO.

6. A pharmaceutical composition according to any preceding claim, wherein the surfactant has a HLB value of 14-16.

7. A pharmaceutical composition according to claim 6 wherein the surfactant is selected from the group Polyoxyl 40 hydrogenated castor oil, Gelucire 44/14, Gelucire 50/13, Labrasol, Acconon MC-8, Acconon C-44, and PEG-35 castor oil..

8. A pharmaceutical composition according to any preceding claim wherein the poorly soluble pharmaceutical agent has a melting point of up to 100°C.

9. A pharmaceutical composition as claimed in any preceding claim wherein the active pharmaceutical agent is present in an amount of from about 1 to about 15 %w/w based on the total weight of the composition.

10. A pharmaceutical composition according to any preceding claim wherein the weight ratio of active pharmaceutical agent to surfactant is in the range 1 : 1.6 to 1 : 1.29.

11. A method of manufacturing the pharmaceutical composition of the present invention comprising the steps of:
(i) melting together the pharmaceutically active agent, the hydrophobic component and the surfactant at a temperature greater than the melting point of the agent to produce a solution;
(ii) dispersing gelatin in water in a ratio of 0.8 : 1 to 1.2 : 1 by weight and allowing it to swell;
(iii) adding the solution produced in step (i) to water which is maintained at a temperature just below its boiling point to form an emulsion;
(iv) adding the swollen gelatin to the emulsion of step (iii) and allowing the gelatin to dissolve.

12. A method as claimed in claim 11 wherein the resultant mixture is processed to produce seamless, spherical beads of size 1.4-1.7mm in diameter.
